# EUROPÄISCHE PATENTANMELDUNG

(11) **EP 1 481 630 A1**
(43) Veröffentlichungstag der Anmeldung: **01.12.2004**
(21) Anmeldenummer: 03007736.6
(22) Anmeldetag: 30.05.2003
(51) Int. Cl.: A61B 1/267

(54) **Spatel für die Laryngoskopie mit beweglichem Vorderteil in Verbindung mit einer eingebauten Optik und abgeschrägtem Lichtaustritt**

(71) Anmelder: Asanus Med. Tech. GmbH, 78579 Neuhausen (DE)
(72) Erfinder: Asanus Med. Tech. GmbH, 78579 Neuhausen (DE)

(57) **Zusammenfassung**

Der Spatel (1) hat einen beweglichen vorderen Teil (6), mit allen möglichen abgewinkelten Lichtaustritten und eine eingebauten starren oder flexiblen Optik (2,3) mit Kaltlichtträgeranschluss, damit am Tracheaeingang genügend Licht zur Verfügung steht und auch eine ungehinderte Sicht auf Tracheaeingang möglich ist. Die Optik (2,3) kann beweglich oder auch fest arretiert sein und auch wieder entfernt werden. Die Gradzahl des Weitwinkels der starren Optik ist unbegrenzt von 0 - 360°. Flexible Optiken aller Art werden ebenfalls mit diesem System eingesetzt.

## Beschreibung

Die bekannten Spatel sind als Radius oder als Gerade ausgebildet, die bei normalen Intubationen ausreichen und ihren Zweck erfüllen. Im vorderen Bereich ist eine Lichtquelle angeordnet, durch die der Rachenraum gut ausgeleuchtet wird.

Schwierig wird es aber, wenn die Stimmlippen mit den herkömmlichen Spateln nicht einsehbar sind. Dies geschieht dadurch, dass die Achse "Tracheaeingang - Oberkieferzähne" durch Instrumente nicht so einstellbar ist, dass der Tracheaeingang sichtbar wird.

Die Erfindung hat die Ziele auch in schwierigen Fällen den Tracheaeingang zu sehen und eine sichere Intuabation zu ermöglichen und das auch in Notfallsituationen, und sie ist in der Handhabung einem Standartspatel ähnlich, so dass eine lange Anlernphase zum Umgang mit diesem Spatel nicht nötig ist.

Wie in der Zeitschrift "Der Anaesthesist" (Jahrgang 96 Heft 12 Seiten 1248 - 1267) beschrieben wird, gibt es verschiedene Methoden den Atemweg zu sichern und auch eine Intubation unter schwierigen Umständen durchzuführen. Aber alle Methoden sind mit einem enormen Materialaufwand als auch mit sehr viel Erfahrung und Übung verbunden.

Der im Patentanspruch angegebenen Erfindung liegt das Problem zugrunde, eine sichere Intubation (auch bei dem schwierigen Atemweg) schnell, an jedem Ort und ohne großen Material- und Ausbildungsaufwand durchführen zu können.

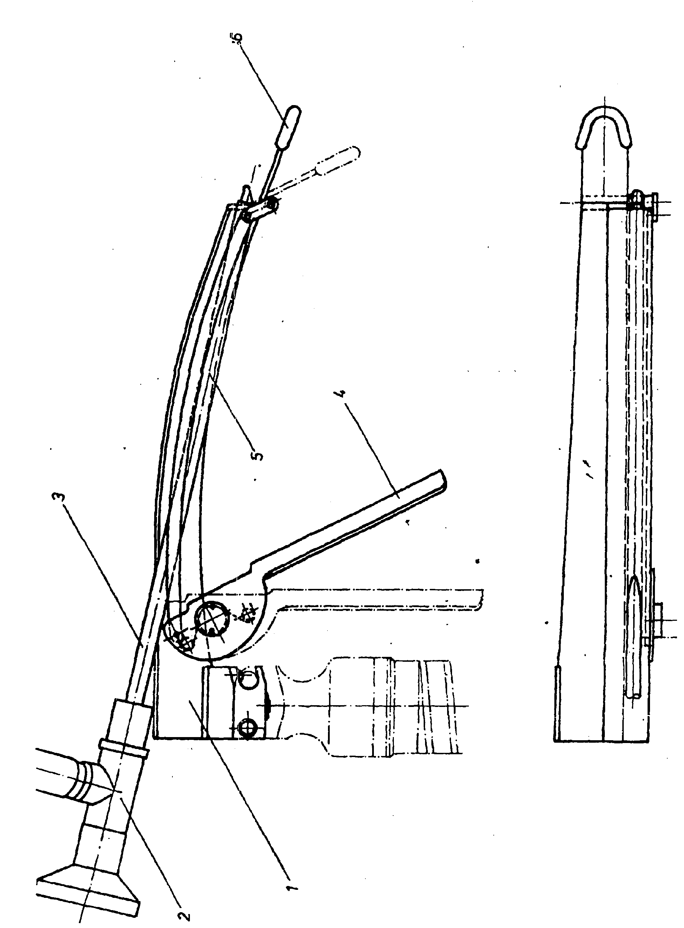

## Patentansprüche

1. Spatel für die Durchführung einer Laryngoskopie und Intubation von Patienten die normal aber insbesondere auch schwer zu intubieren sind, **dadurch gekennzeichnet,**
**dass** der Spatel einen abklappbaren vorderen Teil hat, einen abgeschrägten Lichtaustritt und eine eingebaute Optik mit Kaltlichtträgeranschluss, starr oder flexibel, so dass sehr nahe am Tracheaeingang genügend Licht zur Verfügung steht und auch eine ungehinderte Sicht auf den Tracheaeingang möglich ist. Die Optik kann fest arretiert und auch wieder entfernt werden. Die Gradzahl des Weitwinkels der starren Optik ist on 0 - 75° entsprechend des Optiktyps auszuwählen.
Die Kombination von Kaltlichtspatel und Optik bietet größtmögliche Einsicht in das Indikationsfeld. Durch die bewegliche Spatelführung (siehe Zeichnung Teil 6) vorne mittels dem Spatelzugriff (siehe Zeichnung Teil 4) ist ein kontrolliertes Vorgehen bei der Intubation beim Patienten möglich und eine Traumatisierung durch die runde stärkere Endverarbeitung des Spatels möglich.
